# EUROPEAN PATENT APPLICATION

(11) **EP 1 607 095 A1**
(43) Date of publication of application: **21.12.2005**
(21) Application number: 04723356.4
(22) Date of filing: 25.03.2004
(51) Int. Cl.: A61K 31/715, A61K 31/01, A61K 31/17, A61K 31/722, A61K 31/726, A61K 31/727, A61K 31/728, A61P 17/00, A61P 17/04, A61P 37/08

(54) **ANTIPRURITIC COMPOSITION FOR EXTERNAL USE ON SKIN**

(30) Priority: 25.03.2003 JP 2003083597
(71) Applicant: SEKISUI CHEMICAL CO., LTD., Osaka-shi, Osaka 530 (JP)
(72) Inventor: SHIMIZU, Tatsuo c/o Sekisui Chemical Co., Ltd., Mishima-gun, Osaka 618-8589 (JP); KURIYAMA, Kiyoshi c/o Sekisui Chemical Co., Ltd., Mishima-gun, Osaka 618-8589 (JP)
(74) Representative: Hart Davis, Jason
(86) International application number: PCT/JP2004/004159
(87) International publication number: WO 2004/084918

(57) **Abstract**

It is the object of the present invention to provide a composition which has an antipruritic effect on skin and further exhibits good appearance and feeling of usage.

The present invention relates to an antipruritic composition for external use on skin, which contains a mucopolysaccharide or a derivative thereof in an amount of 0.5 to 20 weight %.

## Description

### TECHNICAL FIELD

The present invention relates to a composition having an excellent antipruritic effect on skin.

### BACKGROUND ART

Conventionally, an antihistamine, a steroid agent and the like has been prescribed as an antipruritic for external use against pruritus of skin, but these drugs have not necessarily had a sufficient antipruritic effect.

Particularly, with respect to xerotic pruritus, the mechanism of its crisis is not clear and therefore there is not a specific treatment currently.

Further, the causes of these prurituses are not the same, and the above drugs may have an effect on pruritus from atopic dermatitis but have little effect on xerotic pruritus, senile skin pruritus and pruritus after receiving kidney-dialysis. The reason for this is that the natures of pruritus are different to one another.

On the other hand, moisturizing agents such as urea, petrolatum and the like are used in order to prevent drying of skin, but these agents cannot improve xerotic pruritus. And, mucopolysaccharides may also be used as a moisturizing agent, but their antipruritic effects are not found at all (Handbook of Cosmetic Ingredients, 2nd Edition, Fragrance Journal Ltd., issued in April, 2002).

### SUMMARY OF THE INVENTION

In view of the above state of the art, it is an object of the present invention to provide a composition having a wide antipruritic effect on various prurituses.

The present inventors made investigations earnestly, and consequently have found that mucopolysaccharides or derivatives thereof exhibit a strong antipruritic effect on skin at the concentration of 0.5 to 20 weight %.

That is, the present invention relates to an antipruritic composition for external use on skin, which contains a mucopolysaccharide or a derivative thereof in an amount of 0.5 to 20 weight %.

The mucopolysaccharide used in the present invention is preferably at least one species selected from the group consisting of hyaluronic acid, chondroitin sulfate, dermatan sulfate, keratan sulfate, heparin, chitin and chitosan and preferably has an average polymerization degree of at least 5 and less than 1200.

The antipruritic composition for external use on skin of the present invention preferably further contains a liquid oil and urea.

### DETAILED DESCRIPTION OF THE INVENTION

Hereinafter, the present invention will be described in detail.

An antipruritic composition for external use on skin of the present invention contains mucopolysaccharides or derivatives thereof.

The above mucopolysaccharides refer to polysaccharides containing hexosamine as a component. The above mucopolysaccharides are not particularly limited but are preferably, for example, at least one species selected from the group consisting of hyaluronic acid, chondroitin sulfate, dermatan sulfate, keratan sulfate, heparin, chitin and chitosan. In addition, the above derivative of mucopolysaccharide refers to a compound formed through a change in a small part in a molecule of the above mucopolysaccharides and include, for example, hyaluronate salt and chondroitin sulfate salt.

The above mucopolysaccharides or derivatives thereof preferably have an average polymerization degree of at least 5 and less than 1200. When the average polymerization degree is less than 5, a sufficient antipruritic effect on skin may not be attained, and when it is 1200 or more, physical properties change and a sufficient antipruritic effect on skin may not also be attained. The average polymerization degree is more preferably at least 10 and less than 1000, and furthermore preferably at least 50 and less than 800.

The content of the above mucopolysaccharides or derivatives thereof is preferably 0.5 to 20 weight %. When this content is less than 0.5 weight %, a sufficient antipruritic effect on skin may not be attained, and when it is more than 20 weight %, a further effect cannot be expected and in addition the composition may become poor in followability on skin because the composition becomes low in solubility and high in viscosity. The content is more preferably 0.5 to 10 weight %, furthermore preferably 0.5 to 8 weight %, particularly preferably 1 to 6 weight %, and the most preferably 1 to 4 weight %.

When chitosan or the derivative thereof is used as the above mucopolysaccharides or derivatives thereof, the antipruritic composition for external use on skin of the present invention preferably contains an acid in order to make the above chitosan or the derivative thereof soluble in water.

As the above acid, any of an inorganic acid and an organic acid can be used. As the above inorganic acid, there are given, for example, hydrochloric acid, phosphoric acid, sulfuric acid and nitric acid. As the above organic acid, there are given, for example, acetic acid, succinic acid, malic acid, lactic acid, butyric acid, fumaric acid, malonic acid, itaconic acid, gluconic acid, glycolic acid, tartaric acid and citric acid. Among others, hydroxy acid, which is a fatty acid having a chemical structure of having a hydroxyl group in carboxylic acid, is preferred. These acids may be used alone or in combination of two or more species of them.

The content of the above acid is preferably 0.1 to 10 weight %. When this content is less than 0.1 weight %, it may be impossible to adequately make the chitosan or the derivative thereof soluble in water, and when it is more than 10 weight %, some locations to which the composition is applied may exhibit skin irritation. The content is more preferably 0.5 to 5 weight %.

The antipruritic composition for external use on skin of the present invention preferably further contains a liquid oil in order to enhance the spread and provide good feeling of usage. The above liquid oil is not particularly limited and includes, for example, hydrocarbons such as squalane and glyceride oils such as triglyceride. Among others, squalane is particularly suitable.

The content of the above liquid oils is preferably 2 to 20 weight % and more preferably 5 to 15 weight %.

The antipruritic composition for external use on skin of the present invention preferably further contains urea.

The above mucopolysaccharides, particularly chitosan, tends to cause a Maillard reaction due to heat in an aqueous solution and may change to brown, but the present inventors have found that by adding urea, chitosan and the like is stabilized and therefore the change to brown can be prevented.

The content of the above urea is preferably 0.1 to 3 weight %. When this content is less than 0.1 weight %, a sufficient effect may not be attained, and when it is more than 3 weight %, there may be cases where an effect of adding urea is not enhanced any more and in addition to this the mucopolysaccharides are separated out and formulation becomes difficult. This content is more preferably 0.1 to 1.5 weight %.

Further, in the antipruritic composition for external use on skin of the present invention, since there is a possibility that a compound having a sulfur atom such as subsulfate may destroy the effect of the present invention, it is preferred not to contain these compounds having a sulfur atom as a component for preventing a Maillard reaction.

The antipruritic composition for external use on skin of the present invention may contain powder, oil content, a surfactant, a thickner, an organic solvent, a plasticizer, dye, pigment, perfume, an antiseptics and an antioxidant to the extent not impairing the object of the present invention.

The form of the antipruritic composition for external use on skin of the present invention is not particularly limited and includes, for example, an ointment, a cream drug and a liquid drug. As the above liquid drug, there are given, for example, a gel agent, a sol agent, an aqueous solution agent, an alcohol agent, a glycerin/glycol agent, an oil agent, a suspension type lotion agent, an emulsion type lotion agent, a varnish agent, a poultice and a spray agent.

A method of producing the antipruritic composition for external use on skin of the present invention is not particularly limited and publicly known methods can be employed.

An application amount of the antipruritic composition for external use on skin of the present invention is not particularly limited depending on kinds or concentrations of effective ingredients or conditions of a body part to which the compositions are applied, but it is preferred that the composition is generally applied at a dose of 0.01 to 10 g once or several times per day. In addition, a method of applying the antipruritic composition for external use on skin of the present invention is not particularly limited and includes a common method of applying an agent for external use on skin, using fingers or utensils such as spatula, a method of directly applying from a pump-, a spray- or a tube-type container and a method of putting a poultice.

### EFFECT OF THE INVENTION

The antipruritic composition for external use on skin of the present invention can mitigate pruritus significantly and is effective against xerotic pruritus, pruritus of dermatitis such as atopic dermatitis and contact dermatitis, senile skin pruritus and pruritus after receiving kidney-dialysis, and further pruritus of eczema, skin irritation, urticaria, heat rash, insect bite and sting, pernio chilblain, exulceration and the like.

### BEST MODE FOR CARRYING OUT THE INVENTION

Hereinafter, the present invention will be described in more detail by way of examples, but the present invention is not limited to these examples.

### (Examples 1 to 20)

Mucopolysaccharides were added to water for injection (produced by Otsuka Pharmaceutical Co., Ltd.) so as to be a content (weight %) shown in Table 1 and the resulting mixture was stirred, dissolving chitosan (average polymerization degree 30 or 300, produced by Wako Pure Chemical Industries, Ltd.) by adding lactic acid (produced by KOZAKAI PHARMACEUTICAL CO., LTD.) only when chitosan is added to the mixture, to obtain the compositions of Examples 1 to 20.

### (Comparative Examples 1 to 5)

Mucopolysaccharides were added to water for injection (produced by Otsuka Pharmaceutical Co., Ltd.) so as to be a content (weight %) shown in Table 1 and the resulting mixture was stirred, dissolving chitosan (average polymerization degree 30 or 300, produced by Wako Pure Chemical Industries, Ltd.) by adding lactic acid (produced by KOZAKAI PHARMACEUTICAL CO., LTD.) only when chitosan is added to the mixture, to obtain the compositions of Comparative Examples 1 to 5.

### (Examples 21 to 46)

Mucopolysaccharides were added to water for injection (produced by Otsuka Pharmaceutical Co., Ltd.) so as to be a content (weight %) shown in Table 1 and the resulting mixture was stirred, dissolving chitosan (average polymerization degree 30 or 300, produced by Wako Pure Chemical Industries, Ltd.) by adding lactic acid (produced by KOZAKAI PHARMACEUTICAL CO., LTD.) only when chitosan is added to the mixture, and to this solution, squalane (produced by MARUHA GROUP INC.) and urea (produced by KOZAKAI PHARMACEUTICAL CO., LTD.) were further added so as to be a content (weight %) shown in Table 1, and the obtained mixture was suspended and emulsified to obtain the compositions of Examples 21 to 46.

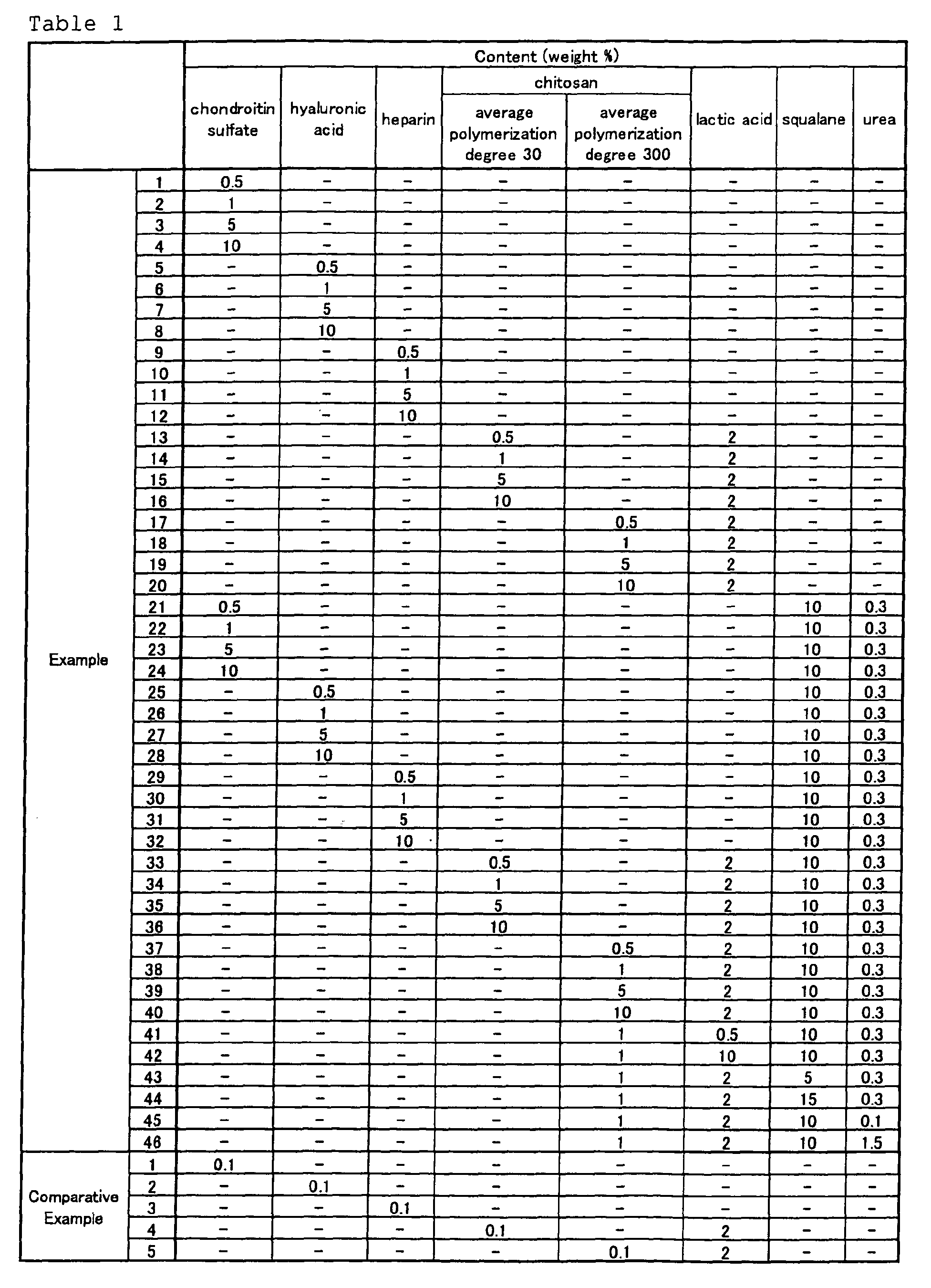

### (Evaluation 1)

Each composition of Examples 2, 6, 10, 14, 18, 22, 26, 30, 34, 38 and Examples 43 to 46 was evaluated based on the following methods. The results are shown in Table 2.

### <Sensory evaluation by healthy individuals>

Sensory evaluations were conducted by healthy individuals on categories of appearance and feeling of usage. Each composition was rated in the above categories at five levels, 5: very good, 4: good, 3: moderate, 2: slightly bad, 1: bad. A higher value means that feeling of usage is better.

**Table 2**

| | | Sensory evaluation | |
|---|---|---|---|
| | | appearance | feeling of usage |
| Example | 2 | 4 | 4 |
| | 6 | 4 | 4 |
| | 10 | 4 | 4 |
| | 14 | 3 | 4 |
| | 18 | 3 | 4 |
| | 22 | 5 | 5 |
| | 26 | 5 | 5 |
| | 30 | 5 | 5 |
| | 34 | 5 | 5 |
| | 38 | 5 | 5 |
| | 43 | 5 | 5 |
| | 44 | 5 | 5 |
| | 45 | 5 | 5 |
| | 46 | 5 | 5 |

As is evident from the results shown in Table 2, the composition exhibited more preferable appearance and feeling of usage by containing urea and liquid oils.

### (Evaluation 2)

Each composition of Examples 1, 4, 6, 11, 14, 17, 20, 38 and Comparative Examples 1 to 3 and 5 was evaluated based on the following methods. The results are shown in Table 3.

### <Evaluation of antipruritic property>

Evaluation of an antipruritic property of agents for external use obtained in the above Examples and Comparative Examples was conducted through a single application by 6 persons (2 males and 4 females) having pruritus. Immediately after applying the agents for external use, these were rated at four levels, 3: remarkably effective, 2: effective, 1: moderate, 0: ineffective, and with respect to each rated value of 3 to 4 persons of 6 persons, the rated value of 2 or more was assumed to be effective and an effective percentage (%) was determined. A higher effective percentage means that an antipruritic property is better.

**Table 3**

| | | Evaluation of antipruritic property | | | | |
|---|---|---|---|---|---|---|
| | | individual value | | | | effective percentage (%) |
| Example | 1 | 3 | 3 | 3 | 2 | 100 |
| | 4 | 3 | 3 | 3 | - | 100 |
| | 6 | 3 | 3 | 3 | 2 | 100 |
| | 11 | 2 | 2 | 3 | 2 | 100 |
| | 14 | 3 | 3 | 3 | 3 | 100 |
| | 17 | 3 | 3 | 3 | 3 | 100 |
| | 20 | 3 | 3 | 3 | 3 | 100 |
| | 38 | 3 | 3 | 3 | 3 | 100 |
| Comparative Example | 1 | 0 | 0 | 1 | 1 | 0 |
| | 2 | 1 | 1 | 0 | 1 | 0 |
| | 3 | 1 | 0 | 0 | - | 0 |
| | 5 | 1 | 0 | 0 | 0 | 0 |

As is evident from the results shown in Table 3, the compositions containing the mucopolysaccharides in an amount of 0.5 weight % or more in Examples exhibited a strong antipruritic effect.

### INDUSTRIAL APPLICABILITY

In accordance with the present invention, it is possible to provide the composition which can realize compatibility between an excellent antipruritic effect on skin and good feeling of usage.

## Claims

1. An antipruritic composition for external use on skin,
which contains a mucopolysaccharide or a derivative thereof in an amount of 0.5 to 20 weight %.

2. The antipruritic composition for external use on skin according to claim 1,
wherein the mucopolysaccharide is at least one species selected from the group consisting of hyaluronic acid, chondroitin sulfate, dermatan sulfate, keratan sulfate, heparin, chitin and chitosan.

3. The antipruritic composition for external use on skin according to claim 1 or 2,
wherein the mucopolysaccharide or the derivative thereof has an average polymerization degree of at least 5 and less than 1200.

4. The antipruritic composition for external use on skin according to 1, 2 or 3,
which further contains a liquid oil.

5. The antipruritic composition for external use on skin according to 1, 2, 3 or 4,
which further contains urea.
